# EUROPEAN PATENT SPECIFICATION

(11) **EP 1 851 858 B1**
(45) Date of publication and mention of the grant of the patent: **08.06.2016**
(21) Application number: 05852938.9
(22) Date of filing: 01.12.2005
(51) Int. Cl.: C12N 5/077, C12N 5/0775, A61L 27/18, A61L 27/36, A61L 27/38, A61L 27/56, D01D 5/00, H03L 7/093, A61K 35/12, C12M 1/12

(54) **SUBSTRATE RECOGNITION BY DIFFERENTIABLE HUMAN MESENCHYMAL STEM CELLS**
SUBSTRATERKENNUNG MITTELS DIFFERENZIERBARER MENSCHLICHER MESENCHYMALER STAMMZELLEN
RECONNAISSANCE DE SUBSTRAT PAR CELLULES SOUCHES MESENCHYMATEUSES HUMAINES DIFFERENCIABLES

(30) Priority: 03.12.2004 US 633233 P
(43) Date of publication of application: 07.11.2007
(73) Proprietor: New Jersey Institute of Technology, Newark, NJ 07102-1982 (US)
(72) Inventor: ARINZEH, Treena, Jersey City, NJ 07303 (US); JAFFE, Michael, Maplewood, NJ 07040 (US); SHANMUGASUNDARAM, Shobana, Lake Hiawatha, NJ 07034 (US)
(74) Representative: Beresford, Keith Denis Lewis
(86) International application number: PCT/US2005/043876
(87) International publication number: WO 2006/068809

(56) References cited:
- SHANMUGASUNDARAM S ET AL: "Applications of electrospinning: Tissue engineering scaffolds and drug delivery system" BIOENGINEERING, PROCEEDINGS OF THE NORTHEAST CONFERENCE - PROCEEDINGS OF THE IEEE 30TH ANNUAL NORTHEAST BIOENGINEERING CONFERENCE 2004 INSTITUTE OF ELECTRICAL AND ELECTRONICS ENGINEERS INC. US, [Online] vol. 30, 17 April 2004 (2004-04-17), - 18 April 2004 (2004-04-18) pages 140-141, XP002557671 Retrieved from the Internet: URL:http://ieeexplore.ieee.org/stamp/stamp .jsp?tp=&arnumber=1300034&isnumber=28882> [retrieved on 2009-11-26]
- LI WAN-JU ET AL: "Electrospun nanofibrous structure: a novel scaffold for tissue engineering." JOURNAL OF BIOMEDICAL MATERIALS RESEARCH 15 JUN 2002, vol. 60, no. 4, 15 June 2002 (2002-06-15), pages 613-621, XP001183765 ISSN: 0021-9304
- BHATTARAI SHANTA RAJ ET AL: "Novel biodegradable electrospun membrane: scaffold for tissue engineering." BIOMATERIALS JUN 2004, vol. 25, no. 13, June 2004 (2004-06), pages 2595-2602, XP004486294 ISSN: 0142-9612
- LI WAN-JU ET AL: "Multilineage differentiation of human mesenchymal stem cells in a three-dimensional nanofibrous scaffold." BIOMATERIALS SEP 2005, vol. 26, no. 25, September 2005 (2005-09), pages 5158-5166, XP025280834 ISSN: 0142-9612
- JIN ET AL.: 'Human bone marrow stromal cell responses on electrospun silk fibroin mats.' BIOMATERIALS vol. 25, 2004, pages 1039 - 1047, XP004472566
- LUU ET AL.: 'Development of a nanostructured DNA delivery scaffold via electrospining of PIGA and PLA-PEG block copolymers.' J CONTROLLED RELEASE. vol. 89, 2003, pages 341 - 353, XP004421368
- SIKAVITSAS ET AL.: 'Mineralized matrix deposition by marrow stromal osteoblasts in 3D perfusion culture increases with increasing fluid shear forces.' PNAS. vol. 100, no. 25, 09 December 2003, pages 14683 - 14688, XP008119430
- PITTENGER M F ET AL: "MULTILINEAGE POTENTIAL OF ADULT HUMAN MESENCHYMAL STEM CELLS", SCIENCE, AMERICAN ASSOCIATION FOR THE ADVANCEMENT OF SCIENCE, WASHINGTON, DC; US, vol. 284, no. 5411, 2 April 1999 (1999-04-02), pages 143-147, XP000867221, ISSN: 0036-8075, DOI: 10.1126/SCIENCE.284.5411.143
- YOSHIMOTO H ET AL: "A biodegradable nanofiber scaffold by electrospinning and its potential for bone tissue engineering", BIOMATERIALS, ELSEVIER SCIENCE PUBLISHERS BV., BARKING, GB, vol. 24, no. 12, 1 May 2003 (2003-05-01), pages 2077-2082, XP004413511, ISSN: 0142-9612, DOI: 10.1016/S0142-9612(02)00635-X
- CHEN G ET AL: "Chondrogenic differentiation of human mesenchymal stem cells cultured in a cobweb-like biodegradable scaffold", BIOCHEMICAL AND BIOPHYSICAL RESEARCH COMMUNICATIONS, ACADEMIC PRESS INC. ORLANDO, FL, US, vol. 322, no. 1, 10 September 2004 (2004-09-10), pages 50-55, XP004526699, ISSN: 0006-291X, DOI: 10.1016/J.BBRC.2004.07.071

## Description

### CROSS REFERENCE TO RELATED APPLICATION

This application claims the benefit of U.S. Provisional Patent Application Serial No. 60/633,233, filed December 3, 2004, and entitled Substrate Recognition by Differentiable Human Mesenchymal Stem Cells.

### FIELD OF THE INVENTION

The present invention relates to compositions and methods comprising a three-dimensional nanofiber matrix of synthetic polymers. The synthetic nanofiber matrix can serve as a scaffold/substrate for delivery of differentiable human mesenchymal cells for tissue engineering applications.

### GOVERNMENT SUPPORT

This work is supported at least in part by grants from the National Science Foundation to Dr. Arinzeh. The government may have certain rights in this invention.

### BACKGROUND OF THE INVENTION

Orthopedic management of lesions to articular cartilage remains a persistent problem for the orthopedist and patient because articular cartilage has a limited intrinsic ability to heal. This has prompted the development of numerous procedures to treat these lesions and to halt or slow the progression to diffuse arthritic changes.

Tissue engineering is the application of principles and methods of engineering and life sciences toward a fundamental understanding and development of biological substitutes to restore, maintain and improve human tissue functions. It may eliminate many of the problems associated with current surgical options. Current tissue engineering methods are aimed at filling the cartilage defects with cells with or without scaffolds to promote cartilage regeneration. Implantation of scaffolds alone leads to a poor quality reparative tissue. Chondrocytes implanted either alone or in combination with a scaffold have failed to restore a normal articular surface, and the hyaline cartilage formed early on in response to chondrocyte-containing scaffolds seems to deteriorate with time. Therefore, improved tissue engineering methods still are needed.

The use of stem cells for tissue engineering therapies is at the forefront of scientific investigation. In the body, adult stem cells often are localized to specific chemically and topologically complex microenvironments, or so-called "niches". Increasing experimental evidence supports the notion that stem cells can adjust their properties according to their surroundings and select specific lineages according to cues they receive from their niche (Xie L, Spradling, AC, "A Niche Maintaining Germ Line Stem Cells In Dropsophila Ovary," Science 290:328 (2000); Fuchs E, Segre J, "Stem Cells: A New Lease On Life," Cell 100: 143-155 (2000), Watt FM, Hogan BLM, "Out Of Eden: Stem Cells And Their Niches," Science 287:1427 (2000)). It follows that in order for an MSC therapy to be successful in the repair of a specific tissue type, the microenvironment of the cells should be designed to relay the appropriate chemical and physical signals to them. Mimicking characteristics of the microenvironment during cartilage development may be a viable approach. During cartilage development, one of the earliest events is pre-cartilage mesenchymal cell aggregation and condensation resulting from cell-cell interaction, which is mediated by cell-cell and cell-matrix adhesion (fibronectin, proteoglycans, and collagens).(DeLise A.M., Fischer L, Tuan RS, "Cellular Interactions And Signaling In Cartilage Development. Osteoarthritis and Cartilage 8: 309-334 (2000)). Type I collagen, the predominant matrix protein present in the early stages of development, is later transformed to Type II collagen by increased cell synthesis during differentiation. (Safronova EE, Borisova NV, Mezentseva SV, Krasnopol'skaya KD, "Characteristics Of The Macromolecular Components Of The Extracellular Matrix In Human Hyaline Cartilage At Different Stages Of Ontogenesis." Biomedical Science 2: 162-168 (1991)). Multiple growth factors and morphogens are also present contributing to the regulation of the differentiation process.

A few studies have demonstrated the use of MSCs for cartilage repair through intra-articular injection and have shown promise.(Murphy M, Fink DJ, Hunziker EB, Barry FP, "Stem Cell Therapy In A Caprine Model Of Osteoarthritis," Arthritis Rheumatism 48: 3464-3474 (2003); Ponticello MS, Schinagel RM, Kadiyala, S, Barry FP, "Gelatin-Based Resorbable Spone As A Carrier Matrix For Human Mesenchymal Stem Cells In Cartilage Regeneration Therapy," J Biomed Materials Res 52: 246-255 (2000)). The MSCs are injected at a high cell density either alone (in saline) or in combination with a gelatinous/hydrogel matrix in order to promote cell-cell aggregation. However, the use of MSCs in combination with biomaterials of varying architectures that may closely mimic the physical architecture of the native extracellular matrix during development to direct chondrogenic differentiation has yet to be investigated.

From a biological viewpoint, almost all human tissues and organs are characterized by well-organized hierarchical fibrous structures through the assembly of nanoscale elements. It is believed that converting biopolymers into fibers and networks that mimic native structures will ultimately enhance the utility of these materials as scaffolds. Nanoscale fibrous scaffolds may provide an optimal template for stem cell growth, differentiation, and host integration.

It is known that cells will attach to synthetic polymer scaffolds leading to the formation of tissue. (Sachlos, E. and Czemuszka, Eur. Cells & Materials 5: 29-40 (2003)). Using fetal bovine chondrocytes maintained in vitro, Li et al. have shown that scaffolds constructed from electrospun three-dimensional nanofibrous poly(ε-capro-lactone) act as a biologically preferred scaffold/substrate for proliferation and maintenance of the chondrocyte phenotype. (Wan-Ju Li, et al., J. Biomed. Mater. Res. 67A: 1105-1114 (2003)).

Most work to develop scaffold materials for tissue engineering, however, has relied on large diameter fibers, which do not mimic the morphological characteristics of the native fibrils of the extracellular matrix.

For example, U.S. Pat. No. 6,472,210, issued to Holy, describes a macroporous polymer scaffold for regenerating bone comprising macropores at least 50% of which have a diameter in the range of 0.5-3.5 mm, a range representative of that found in human trabecular bone, and interconnections as seen in trabecular bone and a process for making the scaffold. The scaffold comprises porous walls consisting of microporous polymer polymer struts defining macropores which are interconnected by macroporous passageways. The microporous struts contain microporous passageways extending through the microporous polymer struts so that macropores on either side of a given strut are in communication through the strut. Cell colonization into the scaffolds required a minimum interconnection size of 0.35 mm and macropore size of 0.7 mm.

U.S. Pat. No. 6,214,369 and U.S. Pat. No. 5,906,934, issued to Grande et al., describe a composition and method for growing new cartilage and or bone in rabbits whereby cells described as mesenchymal stem cells isolated from adult rabbit leg skeletal muscle were cultured on scaffolds of 12-14 µm diameter polyglycolic acid fibers in the form of an intertwined, woven or meshed matrix or a sponge matrix, and the MSC-seeded matrix then implanted into full thickness articular cartilage defects from syngeneic rabbits. Because the cells in this study are not characterized by their cell markers, it is uncertain whether they were stem cells, or at least multipotent cells.

U.S. Pat. No. 6,511,511, and U.S. Pat. No. 6,783,712, issued to Slivka, describe a fiber-reinforced, polymeric implant material comprising a polymeric matrix and a method of making this material for use as a scaffold for tissue implantation. The fibers of the matrix, which are preferably oriented predominantly parallel to each other but may also be aligned in a single plane, are described as preferably about 5 µm to about 50 µm in diameter.

U.S. Pat. No. 6,790,455, issued to Chu describes a cell delivery system comprising viable cells and a fibrous matrix as a carrier physically associated with the cells to contain and release the cells at a controlled rate. In a preferred embodiment, a layered cell storage and delivery system comprises bone cells embedded between two layers of a poly(D,L-lactide (PLLA) or PLGA/ lactide monomer. The first or base layer membrane contains biodegradable nanofibers made from a 40% PLLA or PLGA/lactide monomer by electrospining. Bone cells are deposited on the surface of the membrane after the membrane has been prewet by dipping in a minimum essential medium solution containing 10% fetal bovine serum to increase adhesion between the membrane surface and live bone cells. The cell containing membrane then is covered by a thin top layer of membrane to encapsulate the bone cells near the surface of membrane and to facilitate quick release of the cells from the membrane. The final sandwiched cell/membrane structure described thus consists of three parts: the bottom layer (120-200 µm thick), live cells (15-20 µm thick), and the top layer (several microns thick). While the patent teaches that isolated cells must be undifferentiated and suggests that the cell delivery system when positioned at a desired location for cell delivery to a mammal can guide the development and shape of new tissue, it provides no guidance on how to do so.

The paper by Yoshimoto et al. entitled "A biodegradable nanofiber scaffold by electrospinning and its potential for bone tissue engineering", BIOMATERIALS, vol. 24, no. 12, 1 May 2003, pages 2077-2082 discloses microporous, non-woven poly (ε-caprolactone) scaffolds made by electrostatic fibre spinning. It further discloses seeding the electrospun scaffolds with mesenchymal stem cells derived from the bone marrow of neonatal rats.

The paper by Chen et al. entitled "Chondrogenic differentiation of human mesenchymal stem cells cultured in a cobweb-like biodegradable scaffold", BIOCHEMICAL AND BIOPHYSICAL RESEARCH COMMUNICATIONS, vol. 322. no. 1, 10 September 2004, pages 50-55 discloses a scaffold comprising a knitted mesh of PLGA fibres in the micrometre range and mesenchymal stem cells differentiated into chondrocytes.

Human MSCs used in combination with nanoscale fibrous scaffolds may be an effective potential tissue engineering therapy. Since large diameter fibers do not mimic the morphological characteristics of the native fibrils of the extracellular matrix, biopolymers consisting of fibers and networks that mimic native structures may enhance the utility of these materials. The present invention addresses this problem.

### SUMMARY OF THE INVENTION

In a first aspect, the present invention provides a composition for use in tissue engineering comprising a supporting scaffold for growing isolated differentiable human mesenchymal stem cells, wherein the differentiable human mesenchymal stem cells are differentiated into a mature osteoblast or chondrocyte cell phenotype on the scaffold, as defined is claim 1.

The present invention further provides a method of making an implantable scaffold as defined in claim 7.

The composition and methods of the invention provide nanoscale fibrous scaffolds as a substrate for human mesenchymal stem cells that have the potential to differentiate in situ into mature cell phenotypes. This combination may provide an effective tissue engineering therapy.

### BRIEF DESCRIPTION OF THE FIGURES

Fig. 1 is a diagrammatic representation of the electrospinning equipment used herein.
Fig. 2 shows scanning electron microscope (SEM) images of human MSC loaded PLLA at 14 days in culture containing normal growth media (a) nanofibers; (b) microfibers.
Fig. 3 shows SEM images of human MSC loaded PLGA at 14 days in culture containing normal growth media (a) nanofibers; (b) microfibers.
Fig. 4 shows the growth kinetics of human MSCs grown in standard growth media on polymeric scaffolds.
Fig. 5 is a bar graph showing mineralization of the extracellular matrix as measured by calcium content on days 7 and 11 for cells grown on scaffolds in OS media; *p<0.05.
Fig. 6 is a linear plot showing mineralization of the extracellular matrix as measured by calcium content on days 7 and 11 for cells grown on scaffolds in OS media. *p<0.05.
Fig. 7 shows SEM images of human MSCs cultured in OS medium for 14 days on (a) GSF and (b) PSF scaffolds.
Fig. 8 shows Type II collagen synthesis of cells grown on LF and SF scaffolds in inductive medium TGF-β3.

### DETAILED DESCRIPTION OF THE INVENTION

As used herein, the term "stem cells" refers to undifferentiated cells having high proliferative potential with the ability to self-renew that can migrate to areas of injury and can generate daughter cells that can undergo terminal differentiation into more than one distinct cell phenotype. These cells have the ability to differentiate into various cells types and thus promote the regeneration or repair of a diseased or damaged tissue of interest. The term "cellular differentiation" refers to the process by which cells acquire a cell type. The term "progenitor cell" as used herein refers to an immature cell in the bone marrow that can be isolated by growing suspensions of marrow cells in culture dishes with added growth factors. Progenitor cells are referred to as colony-forming units (CFU) or colony-forming cells (CFC). The specific lineage of a progenitor cell is indicated by a suffix, such as, but not limited to, CFU-F (fibroblastic).

As used herein, the terms "osteoprogenitor cells," "mesenchymal cells," "mesenchymal stem cells (MSC)," or "marrow stromal cells" are used interchangeably to refer to multipotent stem cells that differentiate from CFU-F cells capable of differentiating along several lineage pathways into osteoblasts, chondrocytes, myocytes and adipocytes. When referring to bone or cartilage, MSCs commonly are known as osteochondrogenic, osteogenic, chondrogenic, or osteoprogenitor cells, since a single MSC has shown the ability to differentiate into chondrocytes or osteoblasts, depending on the medium.

The term "chondrocytes" as used herein refers to cells found in cartilage that produce and maintain the cartilaginous matrix. From least to terminally differentiated, the chondrocytic lineage is (i) Colony-forming unit-fibroblast (CFU-F); (ii) mesenchymal stem cell / marrow stromal cell (MSC); (3) chondrocyte. The term "chondrogenesis" refers to the formation of new cartilage from cartilage forming or chondrocompetent cells.

The term "osteoblasts" as used herein refers to cells that arise when osteoprogenitor cells or mesenchymal cells, which are located near all bony surfaces and within the bone marrow, differentiate under the influence of growth factors. Osteoblasts, which are responsible for bone matrix synthesis, secrete a collagen rich ground substance essential for later mineralization of hydroxyapatite and other crystals. The collagen strands to form osteoids: spiral fibers of bone matrix. Osteoblasts cause calcium salts and phosphorus to precipitate from the blood, which bond with the newly formed osteoid to mineralize the bone tissue. Once osteoblasts become trapped in the matrix they secrete, they become osteocytes. From least to terminally differentiated, the osteocyte lineage is (i) Colony-forming unit-fibroblast (CFU-F); (ii) mesenchymal stem cell / marrow stromal cell (MSC); (3) osteoblast; (4) osteocyte. The term "osteogenesis" refers to the formation of new bone from bone forming or osteocompetent cells.

Although the lineage of adipocytes is still unclear, it appears that MSCs can differentiate into two types of lipoblasts, one that give rise to white adipocytes and the other to brown adipocytes. Both types of adipocytes store fat.

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed.

### Example 1. Substrate Recognition by Differentiable Human MSC cells

We have evaluated two commonly used polymeric compositions in the field of tissue engineering, namely poly-L-lactic acid (PLLA) and poly-D,L-lactide glycolide (PLGA) at the nano- and microscale fiber diameter range for their ability to support mesenchymal stem cell attachment. We then compared the morphology and growth characteristics of the attached cells on these substrates.

The term "nanoscale fiber" generally refers to fibers whose diameter ranges from about 1 to about 1000 nanometers. Nanoscale fibers whose average diameter ranges from about 400 to about 500 nanometers are most preferred. The term "microscale fiber" generally refers to fibers whose diameter ranges from about 1 to about 1000 micrometers.

### Polymeric Substrate Materials

The present invention makes use of fibers formed from the biodegradable copolymer of poly L-lactic acid and glycolic acid, 75/25 D,L High IV lactide-co-glycolide (PLGA). PLLA and PLGA were obtained from Alkermes, Inc. As used herein, the term "biodegradable" refers to the ability of a substance or material to break down into harmless substances by the action of living organisms. As used herein, the term "biocompatible material" refers to a material that the body generally accepts without a major immune response, which is capable of implantation in biological systems, for example, tissue implantation, without causing excessive fibrosis or rejection reactions.

### The Electrospinning Process

Electrospinning is a fiber forming technique that relies on charge separation to produce nano- to microscale fibers. A nonwoven matrix of nanofibers was created using the electrospinning technique so that porosity, surface area, fineness and uniformity, diameter of fibers, and the pattern thickness of the matrix could be manipulated. The terms "nonwoven matrix", "nonwoven mesh" or "nonwoven scaffold" are used interchangeably herein to refer to a material comprising a randomly interlaced fibrous web of fibers.

The electrospinning process is affected by varying the electric potential, flow rate, solution concentration, capillary-collector distance, diameter of the needle, and ambient parameters like temperature.

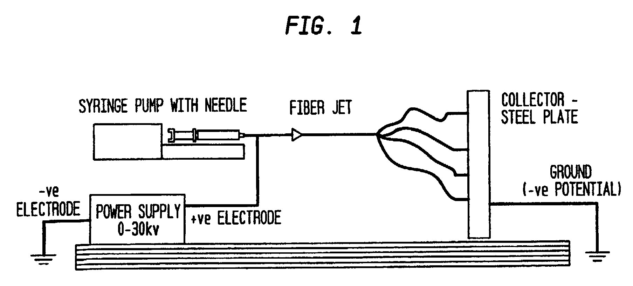

Figure 1 is a diagrammatic representation of the electrospinning setup used herein, which is comprised of a syringe pump containing a 13-20 gauge needle. The syringe pump was mounted on a robotic arm in order to control the splaying of fibers on the collector. An electrically grounded stainless steel plate of dimensions 15 x 30 cm was used as the collector. The syringe pump was filled with the polymer solution, and a constant flow rate of 0.103 ml/min was maintained using the syringe pump. The positive output lead of a high voltage power supply (Gamma High Voltage, Inc.) was attached to the needle, and a 25 kvolt voltage was applied to the solution. The collector-to-needle distance was 20 cm. When the charge of the polymer at increasing voltage exceeded the surface tension at the tip of the needle, the polymer splayed randomly as fibers. These were collected as nonwoven mats on the grounded plate.

### Fabrication Of Tissue Enpineering Scaffolds

In order to make fibers of two different size ranges, scaffolds were fabricated by varying the solution concentration and diameter of the needle. Microfiber scaffolds of PLLA and PLGA were made by electrospinning using a 10% w/w solution concentration of the polymer in methylene chloride using a 13-gauge needle. Nanofiber scaffolds were made by electrospinning using a 5% w/w of polymer solution and a 20-gauge needle.

The fiber diameter of electrospun PLLA and PLGA fibers was characterized using Scanning Electron Microscopy (SEM) according to established methods. Porosity and pore size distribution of the fibers was analyzed by mercury intrusion porosimetry (MIP). Thermal analysis was performed with a TA Model Q100 Differential Scanning Calorimeter (DSC).

By varying the polymer solution concentration and the needle diameter, the electrospinning process yielded very fine fibers with diameters in the nanometer range. MIP results showed that the microfiber and nanofiber scaffolds of PLLA had a porosity of 39% and 47%, respectively. The thermal analysis results show that the electrospinning process as performed herein does not alter the bulk characteristics of glass transition and melting temperatures of these polymers even when the polymer is processed at a high voltage.

The microfiber and nanofiber scaffolds of PLLA and PLGA were made into 3-dimensional 1 mm thick nonwoven mats and sterilized prior to cell seeding.

### Human MSCs

Human MSCs (hMSCs) were prepared as described in Livingston, et al., J. Materials Science: Materials in Med. 14: 211-218 (2003) and in U.S. Patent No. 5,486,359. Bone marrow aspirates of 30-50 ml were obtained from healthy human donors as described. Livingston, et al., J. Materials Science: Materials in Med. 14: 211-218 (2003). Marrow samples were washed with saline and centrifuged over a density cushion of ficoll. The interface layer was removed, washed, and the cells counted. Nucleated cells recovered from the density separation were washed and plated in tissue culture flasks in Dulbecco's Modified Eagle's Medium (DMEM) containing 10% fetal bovine serum ("FBS", HyClone Laboratories, Inc.). Non-adherent cells were washed from the culture during biweekly feedings. Colony formation was monitored for a 14-17 day period. MSC's were passaged when the tissue culture flasks were near confluent. At the end of the first passage, MSCs were enzymatically removed from the culture flask using trypsin-EDTA and replated at a lower density for further expansion. At the end of the second passage, MSC's were either seeded onto scaffolds or cryopreserved until future use.

### Marker Analysis

Human MSC cells were identified as multipotent stem cells based on surface marker characterization, which distinguishes the stem cells from other cell types in the bone marrow, for example white blood cells. Cells expressing CD44 surface antigen and cells from which CD45 and CD34 surface antigens were absent were verified by fluorescence-activated-cell-sorter.

As used herein, "CD44" refers to a common cell surface glycoprotein antigen. CD44 proteins have been implicated in several cellular functions including cell-cell and cell-matrix adhesion, migration, and tumor metastasis (Naor D. et al., Adv. Cancer Res. 71, 241-319 (1997)).

As used herein, "CD34" refers to a novel hematopoietic stem cell antigen selectively expressed on hematopoietic stem and progenitor cells derived from human bone marrow, blood and fetal liver. Yin et al., Blood 90: 5002-5012 (1997); Miaglia, S. et al., Blood 90: 5013-21 (1997). Stromal cells do not express CD34. CD34+ cells derived from adult bone marrow give rise in vitro to a majority of the granulocyte/macrophage progenitor cells (CFU-GM), some colony-forming units-mixed (CFU-Mix) and a minor population of primitive erythroid progenitor cells (burst forming units, erythrocytes or BFU-E). Yeh, et al., Circulation 108: 2070-73 (2003).

As used herein "CD45" refers to a protein tyrosine phosphatase (PTP) located in hematopoietic cells except erythrocytes and platelets. It has several isoforms. The specified expression of the CD45 isoforms can be seen in the various stages of differentiation of normal hematopoietic cells (Virts et al., Immunology 34(16-17) 1119-1197 (1997)).

Our results showed that cells were able to differentiate into three cell types. Osteogenic differentiation was characterized by the expression of alkaline phosphatase activity (as detected by hydrolysis of p-nitrophenylphosphate to p-nitrophenol), by osteocalcin protein expression (quantitated by a competitive immunoassay (Metra Biosystems, Inc), and by mineralization of the extracellular matrix (the amount of calcium present was determined by colorimetric assay). Chondrogenic differentiation was determined by safranin-O staining for glycosaminoglycan and by immunostaining for type II collagen. Adipogenic differentiation was characterized by Oil Red O stain for lipids.

Isolated and subcultured human MSCs were seeded at 1 x 10⁴ cells/cm² onto the microfiber and nanofiber scaffolds in 100 µl in serum-containing medium and maintained at 37°C for 14 days. The term "seeded" refers to the process whereby MSC cells are plated or inoculated onto the scaffolds. Tissue culture plastic was used as a substrate control. Cell proliferation on the scaffolds was assessed using Vybrant®MTT Cell Proliferation Assay Kit (Molecular Probes). The MTT assay involves the conversion of the water soluble MTT (3-(4,5-dimethylthiazol-2-yl)-2,5-diphenyltetrazolium bromide) to an insoluble formazan. The formazan then is solubilized and its concentration measured by colorimetric techniques. Cell morphology was examined by SEM.

### Results

The results of the image analysis of the scaffolds are summarized in table I.

**Table I. Diameter of Nanofiber and Microfiber Scaffolds of PLLA and PLGA**

| | **PLLA** | **PLGA** |
|---|---|---|
| **Microfiber** | Mean=17±7.6µm (n= 4) | Mean=16±7.6µm (n= 4) |
| **Nanofiber** | Mean=400 ±920 nm (n= 4) | Mean=500±880 nm (n= 4) |

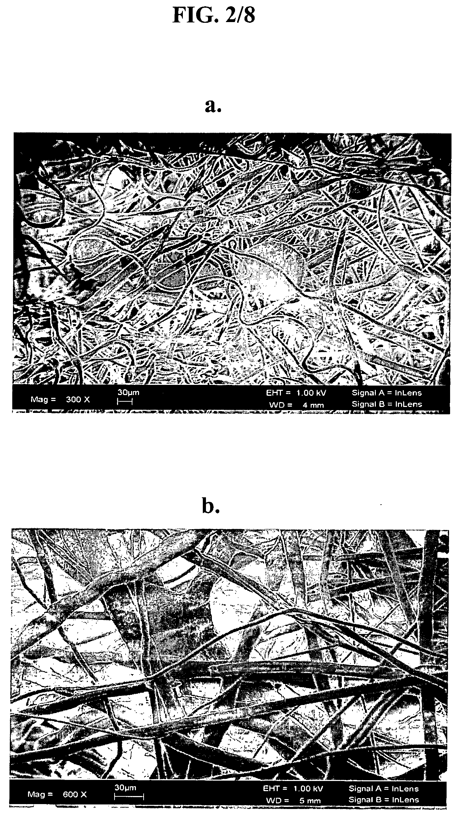

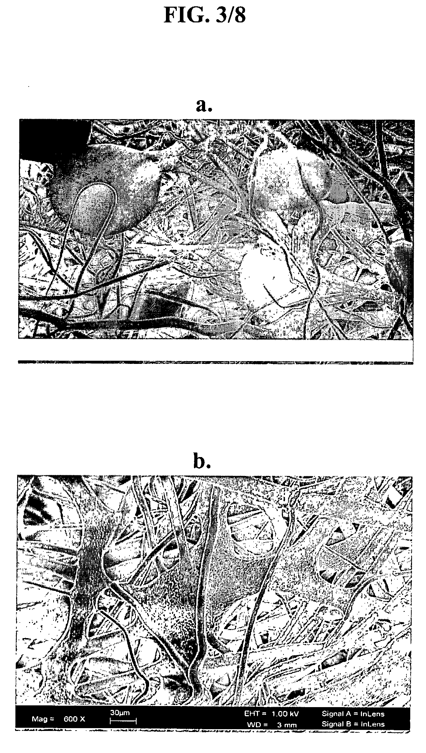

The morphology of the cells on PLLA scaffolds is shown in Fig. 2. Cells were flat and spread out on the microfiber scaffolds, but appeared to be rounded on the nanofiber scaffolds. The hMSCs exhibited a similar morphology on the PLGA scaffolds (Fig. 3). No significant differences in hMSC proliferation were detected between the nano- and microfiber meshes for both PLLA and PLGA. Therefore, the materials used did not alter the growth characteristics of the hMSC cells.

However, striking differences were detected in cell morphology depending on the size of the scaffold fibers. Cells adhered with rounded morphology on the nanofiber scaffolds whereas they appeared flat on the microfiber scaffolds of either material. It is well known that a rounded morphology in vitro is necessary both for chondrogenic differentiation and for maintenance of the chondrocyte phenotype of mature chondrocytes. (Li, et al., J. Biomed. Mater. Res. 67A at 1110). Therefore, the rounded morphology of hMSC cells on nanofiber scaffolds might prove beneficial for MSC chondrogenic differentiation leading, ultimately, when implanted in vivo to treat patients suffering from connective tissue damage, to cartilage formation.

### Example 2. Cell Proliferation

Human MSCs were isolated from adult, human whole bone marrow according to standard techniques and were seeded onto polymer scaffolds having the composition of PLLA or PLGA, each having fiber diameters on the micron scale (LF) or nano scale (SF) and grown in standard growth medium (DMEM, 10% fetal bovine serum, 1% antibiotic/antimycotic) for 14 days. Cell proliferation was assessed using Vybrant's MTT Cell Proliferation Assay Kit (Molecular Probes, Inc.).

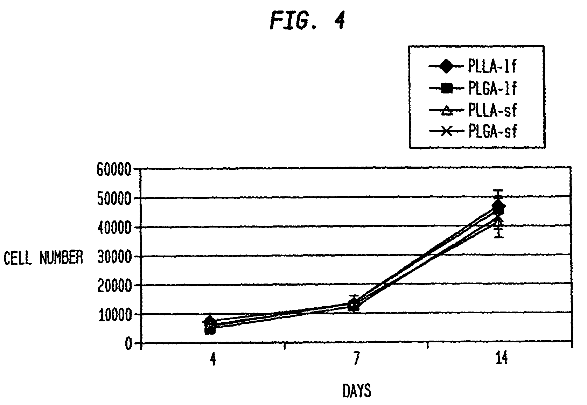

The growth curves for cells seeded onto PLLA micron scale fibers (PLLA-LF),PLLA nano-scale fibers (PLLA-SF), PLGA micron scale fibers (PLGA-LF) and PLGA nano-scale fibers (PLGASF) are shown in Fig. 4. Cells grown on PLLA and PLGA micron scale and nanoscale fibers showed good comparable growth characteristics as measured by the MTT assay. No significant differences in human MSC proliferation were detected between PLLA and PLGA micron and nanoscale fibers.

### Example 3. PLLAlPLGA micron and nano fiber diameter scaffolds support osteogenic differentiation.

Scaffolds were created by the process of electrospinning, and human mesenchymal stem cells were grown on the scaffolds to determine whether PLLA/PLGA micron and nano-sized scaffolds support osteogenic differentiation.

### Materials and Methods

hMSCs were grown in control medium (DMEM, 10% FBS, 1% antibiotic) or osteogenic inducing medium (OS) (Control medium with 100nM dexamethasone, 10mM b-glycerophosphate,0.05mM L-ascorbic acid-2-phosphate) on PLLA or PLGA scaffolds having micron or nano sized fiber diameters.

The four scaffolds, PLLA microfiber ("PLF") scaffolds, PLLA nanofiber scaffolds ("PSF"), PLGA microfiber scaffolds ("GLF"), and PLGA nanofiber scaffolds ("GSF"), were created by electrospinning.

On the day of cell seeding, scaffolds were soaked first in 100% ethanol for 20 minutes, then three times in PBS, 20 minutes each, for sterilization. Scaffolds then were placed into assigned wells of a 96-well microtiter plate (B-D Falcon, Becton-Dickinson, Inc.) for each time point using forceps, and 150 µL of medium containing 10,000 cells were added to each well. The cells were left in the incubator overnight at 37 degrees C to allow cell attachment to the scaffolds. Media were changed the next day so that half of the wells received control medium and the other half received osteogenic induction medium. The media were changed twice a week thereafter.

### Calcium Assay

Mineralization of the extracellular matrix, as an indicator of osteogenic differentiation, was determined by measuring calcium content using a colorimetric assay. 50 µL of 0.5 N HCl were added to 4 different wells. Plates then were incubated at room temperature while the standards (Calcium/Phosphorus Combined Standard, Sigma, Inc.) were prepared for the assays. For the assay, 50 µL of sample was transferred into microcentrifuge tubes. The wells were rinsed with an additional 50 µL of 0.5 N HCl, and this was added to the tube. Tubes were vortexed overnight and then centrifuged for 2 minutes at 3,000 rpm at room temperature. 20 µL of sample was pipetted into a new 96 well plate, and 190 µL of the Working Solution (Cresolphtalin complexone, 0.10 mmol/L, 8-Hydroxyquinoline, 5.2 mmol/L, Polyvinylpyrrolidine, 0.07 mmol/L, 2-Amino-1-methyl proponal, 260 mmol/L, Thermo Electron Calcium Kit) were added to each well. The standards were pipetted in duplicate and consisted of 0, 0.05, 0.1, 0.2, 0.4, 0.6, 0.8, 1.0, 1.5, and 2.0 µg of calcium. The volume of the standard wells was brought up to 190 µL using the Working Solution, and 20 µL of 0.5 N HCl were added to the standard wells. The plate was incubated for 5 minutes at room temperature before being read at 570 nm.

### SEM

Scanning electron micrographs (SEMs) were taken to observe cells growing on the scaffolds and mineralization of the extracellular matrix.

### Results

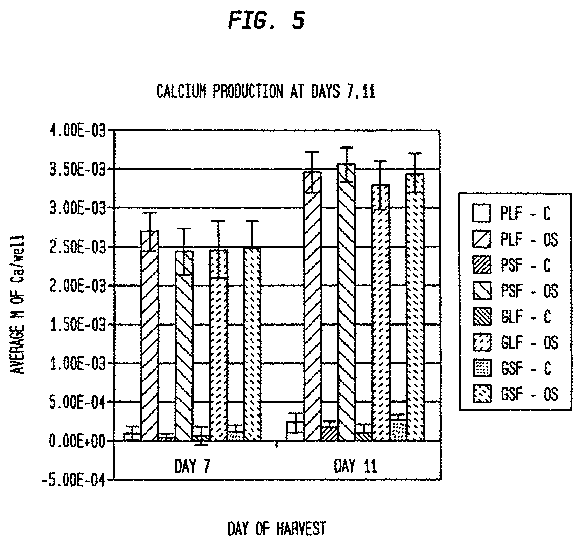

Fig. 5 shows mineralization of the extracellular matrix as measured by calcium content on days 7 and 11 for cells grown on scaffolds in control (C) or OS media. Cells were grown in control medium on PLLA large fiber scaffolds (PLF-C), in control medium on PLLA small fiber scaffolds (PSF-C), in control medium on PLGA large fiber scaffolds (GLF-C), in control medium on PLGA small fiber scaffolds (GSF-C); in osteogenic inducing medium on PLLA large fiber scaffolds (PLF-OS), in osteogenic inducing medium on PLLA small fiber scaffolds (PSF-OS), in osteogenic inducing medium on PLGA large fiber scaffolds (GLF-OS), and in osteogenic inducing medium on PLGA small fiber scaffolds (GSF-OS).

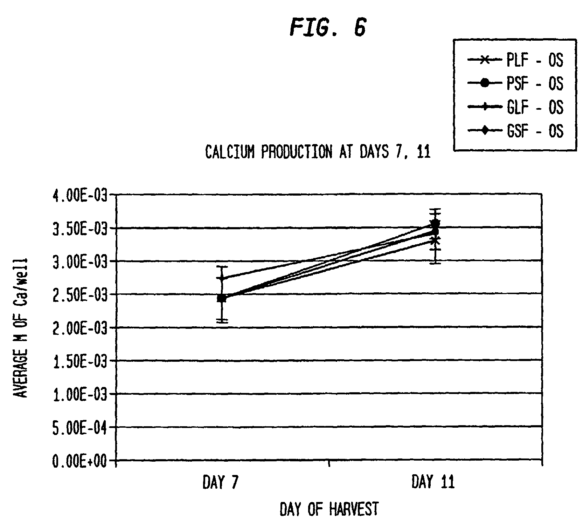

Fig. 5 and 6 show that calcium levels for cells grown on PLLA and PLGA large and small fiber scaffolds in OS media are significantly higher on day 11 than on day 7. This shows positive differentiation in OS medium and that all of the scaffolds can support differentiation successfully. No differences attributable to either fiber size or polymer composition were observed.

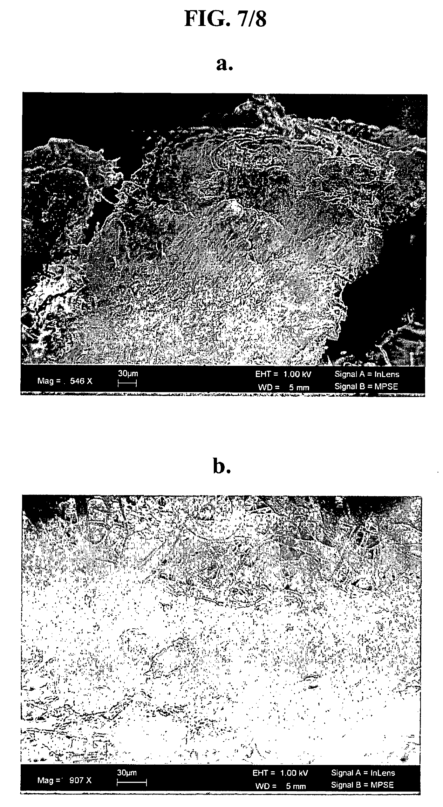

As shown in Fig. 7, SEMs of cells growing on (a) GSF and (b) PSF scaffolds in OS medium for 14 days show a uniform distribution of cells throughout all the scaffolds and an abundant mineralization of the extracellular matrix. Cells form a uniform cell layer, embedded in extracellular matrix, across the surface and interior of the scaffolds.

### Example 4. Chondrogenic differentiation

PLLA and PLGA were made into 1 mm thick non-woven mats of two distinctly different fiber diameters, nanometer (SF) and micrometer (LF), by electrospinning as described in Example 1 and were sterilized prior to cell seeding. SEM, mercury intrusion porosimetry (MIP), and differential scanning calorimetry (DSC) were used to determine fiber diameter and cell morphology; porosity and pore size distribution; and thermal profile, respectively.

To determine the chondrogenic potential of MSCs on LF and SF, MSCs isolated from whole bone marrow and subcultured as described in Example 1 were seeded onto LF and SF scaffolds at a density of 1 x 10⁵ cells/cm². Cells were maintained in chondrogenic induction medium supplemented with TGF-β3 (Cambrex BioScience, Inc.). Type II collagen content on LF and SF scaffolds was assessed with Arthrogen-CIA Capture ELISA Kit (Chondrex, Inc.). A tissue culture polystyrene plate (TCP) was used as control.

The MIP results showed that the PLLA microfiber (LF) and nanofiber (SF) scaffolds had a porosity of 39% and 47% respectively. The DSC results showed that the electrospinning process does not alter the characteristic thermal profile of each polymer even when processed at a high voltage.

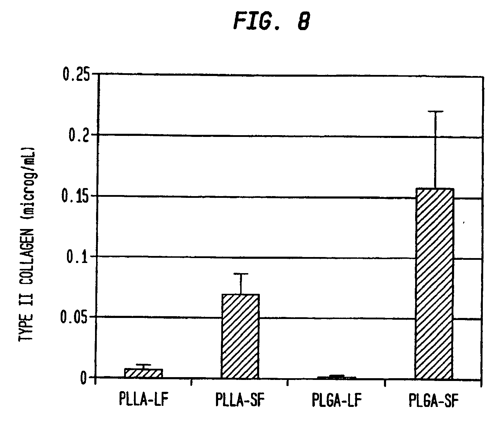

Chondrogenic differentiation occurred on SF fibrous scaffolds at 3 weeks of culture, but was absent on LF fibers, as demonstrated by Type Il collagen synthesis. Fig. 8 shows that type II collagen synthesis by cells grown on PLLA-SF and PLGA-SF scaffolds was significantly greater than synthesis by cells grown on PLLA-LF and PLGA-LF scaffolds.

### Example 5. Cartilaginous tissue repair in a mammalian subject.

Unlike bone, liver, skin and other tissues with high cell turnover rates, cartilage generally is considered to have a limited capacity for self-repair. *See, e.g.,* Laurencin, et al. Ann. Rev. Biomed. Eng'g 1: 19-46, 35 (1999). Cartilage tissue is composed of chondrocytes and an extracellular matrix consisting of proteoglycans, collagen, and water. Chondrocytes are responsible for synthesis and breakdown of collagen and proteoglycans. The collagen fibers provide tear and shear resistance whereas the proteoglycans impart elasticity to cartilage. Because cartilaginous tissue is avascular, has a low oxygen requirement, and has no nerve structures, it may be most amenable to tissue engineering efforts.

The present invention is useful in a partial weight-bearing articular cartilage repair model (Aroen A, et al., "Articular cartilage defects in a rabbit model, retention rate of periosteal flap cover," Acta Orthop. Apr;76(2):220-4 (2005)), to repair a cartilaginous tissue in a mammalian subject. The method comprises the steps of (a) isolating viable differentiable mammalian mesenchymal cells from an autologous mammalian donor; (b) preparing a three-dimensional matrix comprising a nonwoven mesh of fibers to form a cell scaffold; (c) seeding the cell scaffold with the isolated viable differentiable mammalian mesenchymal cells in vitro; (d) growing the differentiable mammalian mesenchymal cells on the cell scaffold in vitro so that the differentiable mammalian mesenchymal cells differentiate into a viable mammalian chondrogenic cell phenotype on the scaffold; and (e) implanting the cell scaffold comprising the viable mammalian chondrogenic cell phenotype.at a site where the cartilaginous tissue of the subject is in need of repair. The differentiable mammalian mesenchymal cells are obtained from mammalian bone marrow. The three-dimensional matrix of fibers in step (a) of the method is formed from a polymeric material. The polymeric material is a biocompatible polymer, preferably poly D,L lactide glycolide or a mixture thereof with poly L-lactic acid.

Unless defined otherwise, all technical and scientific terms used herein have the same meaning as commonly understood by one of ordinary skill in the art to which this invention belongs.

It must be noted that as used herein and in the appended claims, the singular forms "a", "and", and "the" include plural references unless the context clearly dictates otherwise. All technical and scientific terms used herein have the same meaning. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

## Claims

1. A composition for use in tissue engineering comprising:
a supporting scaffold for growing isolated differentiable human mesenchymal stem cells, the supporting scaffold comprising an electrospun three-dimensional matrix of a non-woven mesh of nanofibers formed by electrospinning a solution of poly-D,L-lactide glycolide (PLGA), the matrix being seeded initially with isolated differentiable human mesenchymal stem cells having a CD44+, CD34-, CD45- phenotype, wherein the differentiable human mesenchymal stem cells have differentiated into a mature osteoblast cell phenotype or a mature chondrocyte cell phenotype on the scaffold.

2. The composition according to claim 1, wherein the non-woven mesh of nanofibers further comprises electrospun poly L-lactic acid fibers and wherein the differentiable human mesenchymal stem cells have differentiated into a mature chondrocyte cell phenotype.

3. The composition according to claim 1 wherein the differentiable human mesenchymal stem cells are isolated from human bone marrow.

4. The composition according to claim 1, wherein the seeded differentiable human mesenchymal stem cells are capable of growth throughout the scaffold.

5. The composition according to claim 1, wherein the mature osteoblast cell phenotype mineralizes an extracellular matrix throughout the scaffold.

6. The composition according to claim 5, wherein the extracellular matrix comprises calcium.

7. A method of making an implantable scaffold, the method comprising the steps:
(a) preparing an electrospun three-dimensional matrix of a non-woven mesh of nanofibers formed by electrospinning a solution of PGLA to form a cell scaffold;
(b) seeding the cell scaffold with differentiable human mesenchymal stem cells having a CD44+, CD34-, CD45- phenotype isolated from a human donor; and
(c) growing the differentiable human mesenchymal stem cells on the cell scaffold so that the differentiable human mesenchymal stem cells differentiate into a mature osteoblast cell phenotype or a mature chondrocyte on the scaffold.

8. The method according to claim 7, wherein the differentiable human mesenchymal stem cells used in seeding step (b) are isolated from bone marrow.

9. The method according to claim 7, wherein the non-woven mesh of nanofibers further comprises electrospun poly L-lactic acid fibers and wherein the differentiable human mesenchymal stem cells have differentiated into a mature chondrocyte cell phenotype.

10. The method according to claim 7, wherein in step (c) the mature osteoblast cell phenotype mineralizes an extracellular matrix throughout the three dimensional fiber matrix.

11. The method according to claim 10, wherein the extracellular matrix comprises calcium.

## Patentansprüche

1. Zusammensetzung zur Verwendung bei der Gewebezüchtung, umfassend:
ein Traggerüst zum Züchten von isolierten differenzierbaren menschlichen mesenchymalen Stammzellen, wobei das Traggerüst eine elektro-gesponnene dreidimensionale Matrix aus einem nicht gewebten Geflecht aus Nanofasern umfasst, die durch Elektrospinnen einer Lösung aus Poly-D,L-Lactidglycolid (PLGA) gebildet ist, wobei die Matrix anfänglich mit isolierten differenzierbaren menschlichen mesenchymalen Stammzellen besät wird, die einen Phänotyp CD44+, CD34-, CD45-aufweisen, wobei die differenzierbaren menschlichen mesenchymalen Stammzellen sich in dem Gerüst in einen reifen Osteoblasten-Zellphänotyp oder in einen reifen Chondrocyten-Zellphänotyp differenziert haben.

2. Zusammensetzung nach Anspruch 1, wobei das nicht gewebte Geflecht aus Nanofasern ferner elektro-gesponnene Poly-L-Milchsäure-Fasern umfasst und wobei die differenzierbaren menschlichen mesenchymalen Stammzellen sich in einen reifen Chondrocyten-Zellphänotyp differenziert haben.

3. Zusammensetzung nach Anspruch 1, wobei die differenzierbaren menschlichen mesenchymalen Stammzellen aus menschlichem Knochenmark isoliert sind.

4. Zusammensetzung nach Anspruch 1, wobei die gesäten differenzierbaren menschlichen mesenchymalen Stammzellen imstande sind, durchweg in dem Gerüst zu wachsen.

5. Zusammensetzung nach Anspruch 1, wobei der reife Osteoblasten-Zellphänotyp eine extrazellulare Matrix durchweg in dem Gerüst mineralisiert.

6. Zusammensetzung nach Anspruch 5, wobei die extrazellulare Matrix Kalzium umfasst.

7. Verfahren zum Herstellen eines implantierbaren Gerüsts, wobei das Verfahren die Schritte umfasst:
a) Bereiten einer elektro-gesponnenen dreidimensionalen Matrix aus einem nicht gewebten Geflecht aus Nanofasern, das durch Elektrospinnen einer Lösung aus PGLA zur Ausbildung eines Zellgerüsts gebildet wird,
b) Besäen des Zellgerüsts mit differenzierbaren menschlichen mesenchymalen Stammzellen, die einen von einem menschlichen Spender isolierten Phänotyp CD44+, CD34-, CD45- aufweisen,
c) Züchten der differenzierbaren menschlichen mesenchymalen Stammzellen auf dem Zellgerüst, derart, dass die differenzierbaren menschlichen mesenchymalen Stammzellen in dem Gerüst in einen reifen Osteoblasten-Zellphänotyp oder einen reifen Chondrocyten-Zellphänotyp differenzieren.

8. Verfahren nach Anspruch 7, wobei die bei dem Züchtungsschritt b) verwendeten differenzierbaren menschlichen mesenchymalen Stammzellen aus Knochenmark isoliert werden.

9. Verfahren nach Anspruch 7, wobei das nicht gewebte Geflecht aus Nanofasern ferner elektro-gesponnene Poly-L-Milchsäurefasern umfasst und wobei die differenzierbaren menschlichen mesenchymalen Stammzellen sich in einen reifen Chondrocyten-Zellphänotyp differenziert haben.

10. Verfahren nach Anspruch 7, wobei der reife Osteoblasten-Zellphänotyp beim Schritt c) eine extrazellulare Matrix durchweg in der dreidimensionalen Fasermatrix mineralisiert.

11. Verfahren nach Anspruch 10, wobei die extrazellulare Matrix Kalzium umfasst.

## Revendications

1. Une composition pour une utilisation en ingénierie tissulaire comprenant: un échafaudage support pour la culture de cellules souches mésenchymateuses humaines différentiables isolées, l'échafaudage de support comprenant une matrice tridimensionnelle électrofilée consistant en un maillage non-tissé de nanofibres formées par électrofilage d'une solution d'acide poly-D,L-lactique-glycolique (PLGA), la matrice étant ensemencée initialement par des cellules souches mésenchymateuses humaines différentiables isolées comportant un phénotype CD44+, CD34- ou CD45-, composition dans laquelle, sur l'échafaudage, les cellules souches mésenchymateuses humaines différentiables se sont transformées par différentiation en un phénotype de cellule ostéoblastique mature ou en un phénotype de cellule chondrocytaire mature.

2. La composition selon la revendication 1, dans laquelle le maillage non-tissé de nanofibres comprend en outre des fibres d'acide poly L-lactique électrofilées et dans laquelle les cellules souches mésenchymateuses humaines différentiables se sont transformées par différentiation en phénotype de cellule chondrocytaire mature.

3. La composition selon la revendication 1, dans laquelle les cellules souches mésenchymateuses humaines différentiables sont isolées de la moelle osseuse humaine.

4. La composition selon la revendication 1, dans laquelle les cellules souches mésenchymateuses humaines différentiables ensemencées sont capables de croissance sur l'ensemble de l'échafaudage.

5. La composition selon la revendication 1, dans laquelle le phénotype de cellule ostéoblastique mature minéralise une matrice extracellulaire sur l'ensemble de l'échafaudage.

6. La composition selon la revendication 5, dans laquelle la matrice extracellulaire comprend du calcium.

7. Un procédé d'obtention d'un échafaudage implantable, ce procédé comprenant les étapes:
(a) de préparation d'une matrice tridimensionnelle électrofilée consistant en un maillage non-tissé de nanofibres formées par électrofilage d'une solution de PGLA pour obtenir un échafaudage cellulaire;
(b) d'ensemencement de l'échafaudage cellulaire par des cellules souches mésenchymateuses humaines différentiables comportant un phénotype CD44+, CD34- ou CD45- isolé provenant d'un donneur humain; et
(c) de croissance, sur l'échafaudage cellulaire, des cellules souches mésenchymateuses humaines différentiables de sorte que les cellules souches mésenchymateuses humaines différentiables, se transforment par différentiation, sur l'échafaudage, en phénotype de cellule ostéoblastique mature ou en chondrocyte mature.

8. Le procédé selon la revendication 7, dans lequel les cellules souches mésenchymateuses humaines différentiables utilisées à l'étape d'ensemencement (b), sont isolées de la moelle osseuse.

9. Le procédé selon la revendication 7, dans lequel le maillage non-tissé de nanofibres comprend en outre des fibres d'acide poly L-lactique électrofilées et dans lequel les cellules souches mésenchymateuses humaines différentiables se sont transformées par différenciation en phénotype de cellule chondrocytaire mature.

10. Le procédé selon la revendication 7, dans lequel à l'étape (c) le phénotype de cellule ostéoblastique mature minéralise une matrice extracellulaire dans l'ensemble de la matrice de fibres tridimensionnelles.

11. Le procédé selon la revendication 10, dans lequel la matrice extracellulaire comprend du calcium.
